# EUROPÄISCHE PATENTSCHRIFT

(11) **EP 1 089 669 B1**
(45) Veröffentlichungstag und Bekanntmachung des Hinweises auf die Patenterteilung: **19.03.2008**
(21) Anmeldenummer: 98925375.2
(22) Anmeldetag: 22.06.1998
(51) Int. Cl.: A61B 19/00

(54) **FIDUCIAL MATCHING MITTELS FIDUCIAL-SCHRAUBE**
FIDUCIAL MATCHING BY MEANS OF FIDUCIAL SCREWS
ALIGNEMENT DE REFERENCE A L'AIDE DE VIS DE REPERE

(43) Veröffentlichungstag der Anmeldung: 11.04.2001
(73) Patentinhaber: AO Technology AG, 7002 Chur (CH)
(72) Erfinder: TRAXEL, Doris, CH-4052 Basel (CH); BERGER, Roger, CH-4413 Büren (CH)
(74) Vertreter: Lusuardi, Werther
(86) Internationale Anmeldenummer: PCT/CH1998/000269
(87) Internationale Veröffentlichungsnummer: WO 1999/066853

(56) Entgegenhaltungen:
- EP-A- 0 591 712
- EP-A- 0 832 609
- WO-A-95/15729
- WO-A-97/47240
- DE-A- 19 506 197
- DE-A- 19 536 180
- DE-U- 29 704 393
- US-A- 5 249 581

## Beschreibung

Die Erfindung bezieht sich auf eine Vorrichtung zur Referenzierung zweier an einer in einen Knochen implantierten Fiducial-Schraube angebrachter Referenzpunkte gemäss dem Oberbegriff der unabhängigen Patentansprüche 1 und 5 und auf eine Methode zur Herstellung einer ortsbestimmten Zuordnung von Referenzpunkten gemäss dem Oberbegriff des unabhängigen Patentanspruchs 11.

Im Bereich der Computer Assisted Surgery stellt sich häufig die Aufgabe, dass beispielsweise ein Computer-Tomogramm der zu behandelnden Knochen oder Körperteile als Vorlage für den chirurgischen Eingriff dient. Dabei kann es notwendig sein, mittels einer Positionserfassungseinrichtung für die chirurgischen Instrumente einen bestimmten Punkt am Knochen oder Körperteil zu identifizieren und mit dem identischen Punkt auf dem Computer-Tomogramm bezüglich der Position zu vergleichen.

Um eine möglichst geringe Schädigung der Weichteile um den Knochen zu erreichen, soll der Knochen oder beispielsweise auch ein Wirbelsäulensegment ohne grossflächiges Freilegen der zu behandelnden Teile operiert werden (Minimal Invasive Technik). Dazu eignet sich Computer Assisted Surgery. Zur Registrierung des Koordinatensystems des Patienten im Operationssaal, welches durch die dynamische Referenzbasis des Positionserfassungssystems gegeben ist, und dem Koordinatensystem der vorgängig erfassten tomographischen Bilddaten muss eine Koordinatentransformation durchgeführt werden, welche als Matching bezeichnet wird.

Eine ausführlichere Beschreibung zur Computer Assisted Surgery mit Angabe eines zur erwähnten Koordinatentransformation geeigneten Matching Algorithmusses findet sich in:
L.-P. Nolte et al. Clinical evaluation of a system for precision enhancement in spine surgery Clinical Biomechanics, 1995, Vol. 10, No. 6 pp 293-303

Eine Möglichkeit das Koordinatensystems des Patienten im Operationssaal mit dem Koordinatensystem auf dem Bild des Patienten zu vergleichen, liegt in der Anwendung von mechanischen Scanning-geräten, wie sie beispielsweise in der US 5,383,454 BUCHHOLZ beschrieben werden. Diese bekannten Methoden sind jedoch ausserordentlich zeitraubend und von ihrer Genauigkeit her heute überholt.

Eine weitere Möglichkeit zum Matchen des Koordinatensystem des Patienten im Operationsraum und dem Koordinatensystem des Bildes besteht aus der Identifikation von vorherbestimmten Punkten mit der Hilfe von anatomischen Referenzpunkten. Auch diese Methode ist wegen der geringen Übersicht bei wenig freigelegten Körperteilen schwierig. Oft wird auch ein Endoskop benötigt.

Eine Vorrichtung zur präoperativen Bestimmung der Positionsdaten von Endoprothesenteilen eines mittleren Gelenkes relativ zu den das mittlere Gelenk ausbildenden Knochen wird in der DE-U-29 704 393 AESCULAP offenbart. Diese bekannte Vorrichtung beinhaltet eine Messeinrichtung zur Bestimmung der Lage von Markierungselementen innerhalb eines drei-dimensionalen Koordinatensystems, eine Datenverarbeitungsantage und je ein Markierungselement für die beiden das Gelenk ausbildenden Knochen. Diese Markierungselemente bestehen je aus einem in einen Knochen einschraubbaren Fuss in Form einer Knochenschraube und einem T-förmigen Aufsetzkörper, welcher vier voneinander beabstandete Marker umfasst.

In einer anderen Methode zum Registrieren der Koordinatensysteme werden zur eindeutigen Identifikation von Referenzpunkten sogenannte Fiducial-Implantate verwendet. Eine solche Methode und dazu geeignete Vorrichtung ist in der US 4,945,914 ALLEN beschrieben. Die Methode beinhaltet die Implantation von mindestens 4 Fiducials in einer räumlichen Beziehung.

Alle diese bekannten Methoden zeigen den Nachteil, dass sie relativ zeitaufwendig sind.

Hier will die Erfindung Abhilfe schaffen. Der Erfindung liegt die Aufgabe zugrunde, eine Vorrichtung zu schaffen, welche die Herstellung einer ortsbestimmten Zuordnung von an Fiducial-Schrauben angebrachten Referenzpunkten zwischen deren Positionen im Koordinatensystem des realen Körpers und der Positionen der identischen Referenzpunkte im Koordinatensystem des Bildes ermöglicht und dabei nur eine geringe Schädigung der um den oder die Knochen liegenden Weichteile erfordert (Minimal Invasive Technik). Kernpunkt der Vorrichtung ist die Definition zweier Referenzpunkte an einer Fiducial-Schraube, womit dann das Verfahren nur zwei implantierte Fiducial-Schrauben benötigt. Das Bild kann auch ein Röntgenbild oder digital gespeichertes Computer-Tomogramm sein.

Die Erfindung löst die gestellte Aufgabe mit einer Vorrichtung zur Referenzierung zweier an einer in einen Knochen implantierten Fiducial-Schraube angebrachter Referenzpunkte, welche die Merkmale des Anspruchs 1 aufweist.

Bei der erfindungsgemässen Vorrichtung wird ein mit Markiermitteln ausgestatteter Pointer in eine bezüglich einer mit festen Referenzpunkten versehenen Fiducial-Schraube räumlich genau definierte Lage und Orientierung gebracht. Der eine Referenzpunkt ist durch die Spitze der Fiducial-Schraube bestimmt, während der zweite Referenzpunkt durch den Schraubenkopf festgelegt ist. Die Positionierung des Pointers erfolgt durch Einbringen eines passgenauen Zapfens am vorderen Ende des Pointers in eine entsprechende Öffnung am Schraubenkopf der Fiducial-Schraube. Die Öffnung im Schraubenkopf der Fiducial-Schraube und somit auch der Zapfen liegen dann konzentrisch zur Längsachse der Fiducial-Schraube, wobei der Zapfen wiederum konzentrisch am Pointer angebracht ist. Der Pointer und die Fiducial-Schraube liegen somit in einer bezüglich den an der Fiducial-Schraube angebrachten Referenzpunkten definierten Achse. In Richtung dieser Längsachse ergibt sich eine definierte Position des Pointers dadurch, dass der an den Zapfen anschliessende Schaft des Pointers einen grösseren Querschnitt als der Zapfens aufweist und somit auf dem Schraubenkopf ansteht. Bei einer in einem Koordinatensystem bekannten Position der Markiermittel lässt sich somit die Position der an der Fiducial-Schraube angebrachten Referenzpunkte im gleichen Koordinatensystem erfassen. Die Markiermittel können elektromagnetische oder akustische Wellen abgebende oder detektierende Sensoren oder Sender sein. Je nach Beschaffenheit der Sensoren oder Sender kann die Position der Markiermittel mit einer entsprechenden Positionserfassungseinrichtung vermessen werden.

Die Positionserfassungseinrichtung kann auch Teil eines Computer Assisted Surgery Systems (CAS) sein, welches unter anderem einen Computer mit Bildverarbeitungssoftware umfassen kann.

Die erfindungsgemässe Methode zur Herstellung einer ortsbestimmten Zuordnung von Referenzpunkten basiert darauf, dass die in einen Knochen implantierten Fiducial-Schrauben auf einem beispielsweise mittels Computer-Tomographie erstellten digitalen dreidimensionalen Bild oder einem gewöhnlichen Röntgenbild des Knochens erkennbar sind. Werden die Referenzpunkte an den Fiducial-Schrauben nun auf dem Bild bezüglich eines entsprechenden Koordinatensystems vermessen und die Referenzpunkte an den im realen Körper angebrachten Fiducial-Schrauben bezüglich eines anderen Koordinatensystems mittels der erfindungsgemässen Vorrichtung vermessen, so ermöglicht eine Zuordnung zwischen den Positionen der Referenzpunkte auf dem Bild und denjenigen am realen Körper, dass jeder beliebige Punkt auf dem Bild durch eine Koordinatentransformation dem entsprechenden Punkt am realen Knochen zugeordnet werden kann und umgekehrt. Bei der erfindungsgemässen Methode mit Anwendung der erfindungsgemässen Vorrichtung genügen zwei in den Körper implantierte Fiducial-Schrauben zum Registrieren oder Matchen der Koordinatensysteme.

Die Herstellung der ortsbestimmten Zuordnung von an Fiducial-Schrauben angebrachten Referenzpunkten zwischen deren Positionen im Koordinatensystem des realen Körpers und der Positionen der identischen Referenzpunkte im Koordinatensystem des Bildes wird üblicherweise als Fiducial-Matching bezeichnet.

Weitere vorteilhafte Ausgestaltungen der Erfindung sind in den abhängigen Ansprüchen gekennzeichnet.

Die durch die Erfindung erreichten Vorteile sind im wesentlichen darin zu sehen, dass dank der erfindungsgemässen Vorrichtung unter Anwendung der erfindungsgemässen Methode das Implantieren von zwei Fiducial-Schrauben für das Fiducial-Matching des Bild-Koordinatensystems und des realen Koordinatensystems genügt und das Knochensegment, in welches die Fiducial-Schrauben implantiert sind, nur in geringem Masse freigelegt werden muss (Minimal Invasive Technik).

Die Erfindung und Weiterbildungen der Erfindung werden im folgenden anhand der teilweise schematischen Darstellungen mehrerer Ausführungsbeispiele noch näher erläutert.

Es zeigen:
Fig. 1 eine perspektivische Darstellung einer Ausführungsform der erfindungsgemässen Vorrichtung; und
Fig. 2 eine teilweise geschnittene Seitenansicht einer Ausführungsform der erfindungsgemässen Vorrichtung.

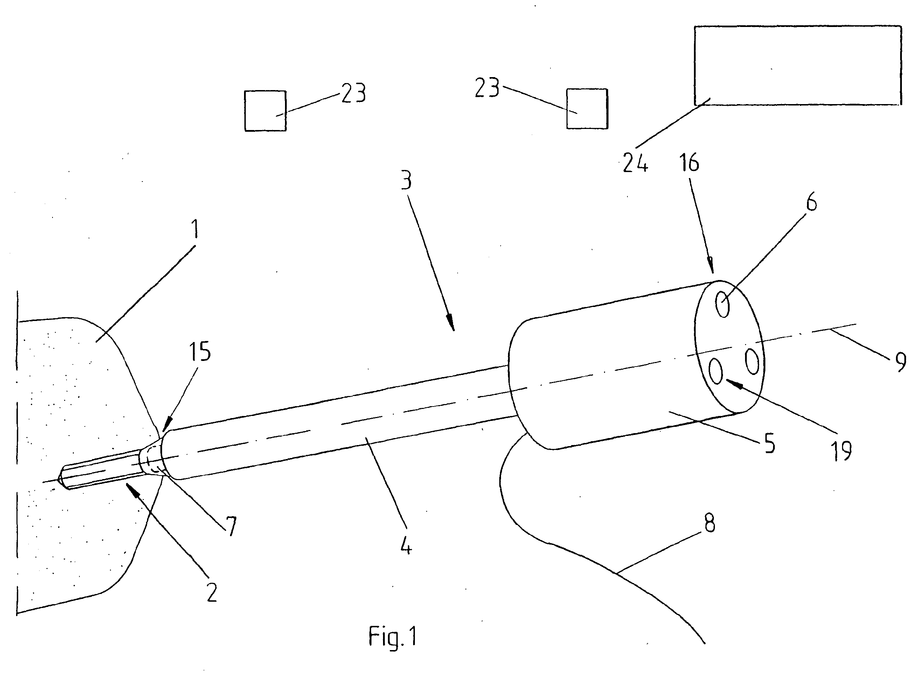

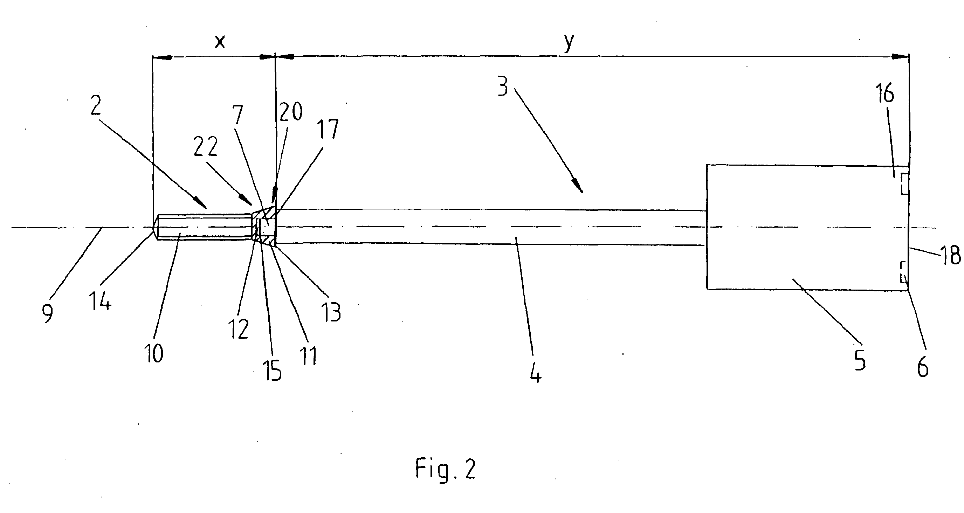

Die in Fig. 1 und 2 dargestellte Ausführungsform der erfindungsgemässen Vorrichtung umfasst eine Fiducial-Schraube 2 mit einer Längsachse 9 und einen Pointer 3 mit einem vorderen Ende 15, einem hinteren Ende 16, einem zylindrischen Schaft 4 und einem Handgriff 5. Am vorderen, dem Handgriff 5 gegenüberliegenden Ende 15 des Pointers 3 ist ein zylindrischer Zapfen 7 angebracht, welcher konzentrisch passgenau in die am Schraubenkopf 22 der Fiducial-Schraube 2 angebrachte Öffnung 12 zur Aufnahme eines zum Ein- und Ausdrehen der Fiducial-Schraube 2 dienenden Instrumentes einfügbar ist. Der Zapfen 7 weist einen kleineren Querschnitt auf als der Schaft 4 des Pointers 3, wodurch die axiale Position des eingeführten Pointers 3 exakt definiert ist, da die Schulter 17 auf dem vorzugsweise senkrecht zur Längsachse 9 stehenden hinteren Ende 13 der Fiducial-Schraube 2 aufliegt. Dabei ist diese konzentrisch zur Längsachse 9 verlaufende Öffnung 12 im Schraubenkopf 22 der Fiducial-Schraube 2 mit einem Innengewinde oder mit einem Innensechskant versehen. Ist diese Öffnung 12 mit einem Innengewinde versehen, kann der Zapfen 7 des Pointers 3 mit einem Aussengewinde versehen sein, wodurch eine lösbare, feste Verbindung zwischen dem Pointer 3 und der Fiducial-Schraube 2 herstellbar ist. Durch dieses konzentrische Zusammenfügen des Pointers 3 mit der Fiducial-Schraube 2 fällt die Längsachse 9 des Pointers mit der Längsachse 9 der Fiducial-Schraube 2 zusammen, wodurch die Richtung des zylindrischen Pointers 3 bezüglich der Fiducial-Schraube 2 definiert ist. Am hinteren Ende 16 des Pointers 3 sind drei Light Emitting Dioden (LED) 6 so angebracht, dass diese in einer Ebene 18 liegen, welche am hinteren Ende 16 des Pointers 3 sein kann und senkrecht zur Längsachse 9 des Pointers 3 steht. Die Light Emitting Dioden 6 liegen nicht auf einer Linie.

Gemäss Fig. 2 sind die Länge (x) zwischen dem vorderen, den einen Referenzpunkt bildenden Ende 14 und dem den zweiten Referenzpunkt bildenden Schraubenkopf 22 der Fiducial-Schraube 2 und die Länge (y) zwischen der Schulter 17 und der Ebene 18, in welcher die Light Emitting Dioden 6 liegen, genau festgelegt. Durch die Kenntnis der Position der Light Emitting Dioden 6 bezüglich eines Koordinatensystems im Raum, die koaxiale und konzentrische Ausrichtung der Längsachse 9 des Pointers 3 und der Längsachse 9 der Fiducial-Schraube 2 und die axial definierte Position des Pointers 3 gegenüber der Fiducial-Schraube 2 mittels der auf dem Schraubenkopf 22 anstehenden Schulter 17 am Schaft 4 des Pointers 3 lassen sich somit die Orte der durch das vordere Ende 14 und den Schraubenkopf 22 definierten Referenzpunkte in demselben Koordinatensystem im Raum ermitteln.

Die Light Emitting Dioden 6 können gemäss der in Fig. 1 gezeigten Ausführungsform der erfindungsgemässen Vorrichtung mittels eines Kabels 8 mit Strom versorgt werden. Andere Arten der Stromversorgung wie Batterien oder ein Akkumulator sind ebenfalls denkbar. Im Falle, dass die mindestens drei nicht kolinear angeordneten, elektromagnetische oder akustische Wellen abgebenden Mittel 19 wie bei der in Fig. 1 und 2 dargestellten Ausführungsform der erfindungsgemässe Vorrichtung Light Emitting Dioden 6 sind, kann die räumliche Ortsvermessung der Light Emitting Dioden 6 bezüglich eines Koordinatensystems mittels einer der im Handel erhältlichen, optischen Positionserfassungsgeräte erfolgen. Ein solches optisches Positionserfassungsgerät ist unter dem Namen Optotrak^{™} erhältlich.

Ist die Fiducial-Schraube 2 gemäss Fig. 1 in einen Knochen eingeschraubt, lassen sich die Orte der durch das vordere Ende 14 und den Schraubenkopf 22 definierten Referenzpunkte der realen Fiducial-Schrauben 2 mit den Orten derselben, auf einem zu einem anderen Zeitpunkt beispielsweise mittels Computer-Tomographie aufgenommenen, dreidimensionalen Bild abgebildeten Referenzpunkte in den beiden jeweils massgebenden Koordinatensystemen matchen respektive registrieren.

Eine Ausführungsform der erfindungsgemässen Vorrichtung kann zusätzlich eine Positionserfassungseinrichtung (24) umfassen, welche im Raum mindestens zwei die von den Mitteln (19) abgegebenen elektromagnetischen oder akustischen Wellen detektierende Sensoren (23) einschliesst.

## Patentansprüche

1. Vorrichtung zur Referenzierung zweier an einem in einen Knochen implantierten Fiducial-Implantat (2) angebrachter Referenzpunkte (11;14), wobei die Vorrichtung
A) ein in einen Knochen (1) einbringbares Fiducial-Implantat (2) mit einer Längsachse (9), einem Schaft (10) und einem Kopf (22); und
B) einen mit mindestens drei nicht kolinear angeordneten, elektromagnetische oder akustische Wellen abgebenden oder detektierenden Markiermitteln (19) versehenen Pointer (3) mit einem vorderen Ende (15), einem hinteren Ende (16) und einer Längsachse (9) umfasst, wobei
C) der Pointer (3) an seinem vorderen Ende (15) und das Fiducial-Implantat (2) an seinem Kopf (22) mit Mitteln (20) versehen sind, die gestatten, dass der Pointer (3) und das Fiducial-Implantat (2) zur Längsachse (9) konzentrisch und in Richtung der Längsachse (9) definiert zusammenfügbar sind,
D) durch die bekannte Länge (x) des Fiducial-Implantates (2), die bekannte Länge (y) des Pointers (3) und die unter C) erwähnten Mittel (20) ein definierter örtlicher Zusammenhang zwischen den Referenzpunkten (11;14) an dem Fiducial-Implantat (2) und den Markiermitteln (19) am Pointer (3) herstellbar ist; **dadurch gekennzeichnet, dass**
E) das Fiducial-Implantat (2) eine Fiducial-Schraube ist, deren Kopf (22) eine mit einem Innengewinde oder einem Innensechskant versehene Öffnung (12) zur Aufnahme eines zum Ein- und Ausdrehen der Fiducial-Schraube dienenden Instrumentes umfasst,
und **dass**
F) der Pointer (3) an seinem vorderen Ende (15) mit einem Zapfen (7) und einer daran anschliessenden Schulter (17) versehen ist und dieser Zapfen (7) koaxial zur Längsachse (9) passgenau in der Öffnung (12) aufnehmbar ist und die Schulter (17) beim Einbringen des Pointers (3) in diese Öffnung (12) auf dem Schraubenkopf (11) ansteht.

2. Vorrichtung nach Anspruch 1, **dadurch gekennzeichnet, dass** die Markiermittel (19) elektromagnetische oder akustische Wellen abgeben und die Vorrichtung zusätzlich eine Positionserfassungseinrichtung (24) umfasst, welche im Raum mindestens zwei die von den Markiermitteln (19) abgegebenen elektromagnetischen oder akustischen Wellen detektierende Sensoren (23) einschliesst.

3. Vorrichtung nach Anspruch 1, **dadurch gekennzeichnet, dass** die Markiermittel (19) elektromagnetische oder akustische Wellen detektieren und die Vorrichtung zusätzlich eine Positionserfassungseinrichtung (24) umfasst, welche im Raum mindestens zwei Sender (23), die die von den Markiermitteln (19) zu detektierenden elektromagnetischen oder akustischen Wellen emittieren.

4. Vorrichtung nach einem der Ansprüche 1 bis 3, **dadurch gekennzeichnet, dass** die Markiermittel (19) in einer senkrecht zur Längsachse (9) stehenden Ebene (18) am Pointer (3) zwischen der Schulter (17) und dem hinteren Ende (16) angebracht sind.

5. Vorrichtung nach einem der Ansprüche 1 bis 3, **dadurch gekennzeichnet, dass** die Markiermittel (19) in einer die Längsachse enthaltenden Ebene (18) am Pointer (3) zwischen der Schulter (17) und dem hinteren Ende (16) angebracht sind.

## Claims

1. A device for referencing two reference points (11; 14) at a fiducial implant (2) in a bone, this device comprising
a) a fiducial implant (2) insertable into a bone (1) and having a longitudinal axis (9), a shaft (10) and a head (22), and
b) a pointer (3) fitted with at least three non-collinearly arranged marking means (19) emitting electromagnetic or acoustic waves, said pointer (3) having a front end (15), a rear end (16) and a longitudinal axis (9), whereby
c) the pointer (3) is fitted at its front end (15) and the fiducial implant (2) is fitted at its head (22) with means (20) allowing to assemble the pointer (3) and the fiducial implant (2) concentrically to the longitudinal axis (9) and in defined manner in the direction of the longitudinal axis (9),
d) a defined spatial relationship between the reference points (11; 14) at the fiducial implant (2) and the marking means (19) at the pointer (3) can be derived from the known length (x) of the fiducial implant (2), the known length (y) of the pointer (3) and the means (20) cited in (c),
**characterized in that**
e) the fiducial implant (2) is a fiducial screw, where the head (22) comprises an opening (12) which is provided with an internal thread or a hexagon socket for receiving an instrument apt to screw in or out the fiducial screw; and that
f) the pointer (3) is fitted at its front end (15) with a pin (7) and an adjoining shoulder (17) and **in that** this pin (7) can be received snugly in the opening (12) and that, upon the pointer (3) being inserted into this opening (12) the shoulder (17) abuts the screw head (11).

2. Device as claimed in claim 1, **characterized in that** the marking means (19) emit electromagnetic or acoustic waves and that this device additionally comprises a position finding device (24) comprising at least two sensors (23) in space detecting the electromagnetic or acoustic waves emitted by the marking means (19).

3. Device as claimed in claim 1, **characterized in that** the marking means (19) detect electromagnetic or acoustic waves and that the device additionally comprises a position finding device (24) including at least two emitters (23) in space emitting the electromagnetic or acoustic waves to be detected by the marking means (19).

4. Device as claimed in one of claims 1 through 3, **characterized in that** the marking means (19) are configured at the pointer (3) in a plane (18) perpendicular to the longitudinal axis (9) and between the shoulder (17) and the rear end (16).

5. Device as claimed in one of claims 1 through 3, **characterized in that** the marking means (19) are configured at the pointer (3) in a plane (18) containing the longitudinal axis (9), between the shoulder (17) and the rear end (16).

## Revendications

1. Dispositif de référencement de deux points de référence (11 ; 14) situés sur un implant de repérage (2) implanté dans un os (1), dans lequel le dispositif comprend
A) un implant de repérage (2), pouvant être inséré dans un os (1), comprenant un axe longitudinal (9), une tige (10) et une tête (22) ; et
B) un pointeur (3), pourvu d'au moins trois moyens de marquage (19) disposés de façon non colinéaire, émettant ou détectant des ondes électromagnétiques ou acoustiques, comprenant une extrémité avant (15), une extrémité arrière (16) et un axe longitudinal (9), dans lequel
C) le pointeur (3), au niveau de son extrémité avant (15), et l'implant de repérage (2), au niveau de sa tête (22), sont pourvus de moyens (20) qui permettent d'assembler le pointeur (3) et l'implant de repérage (2) de manière concentrique par rapport à l'axe longitudinal (9) et de manière définie dans la direction de l'axe longitudinal (9),
D) grâce à la longueur connue (x) de l'implant de repérage (2), à la longueur connue (y) du pointeur (3) et aux moyens (20) mentionnés en C), il est possible d'établir un rapport spatial défini entre les points de référence (11 ; 14) situés sur l'implant de repérage (2) et les moyens de marquage (19) situés sur le pointeur (3) ;
**caractérisée en ce que**
E) l'implant de repérage (2) est une vis de repère dont la tête (22) comprend une ouverture (12) taraudée ou à six pans creux destinée à recevoir un instrument servant au vissage et au dévissage de la vis de repère,
et **en ce que**
F) le pointeur (3) est pourvu, au niveau de son extrémité avant (15), d'un tenon (7) et d'un épaulement (17) adjacent à celui-ci, et ce tenon (7) peut être logé dans l'ouverture (12) avec un ajustement précis, coaxialement à l'axe longitudinal (9), et l'épaulement (17) s'appuie contre la tête de vis (11) lors de l'insertion du pointeur (3) dans cette ouverture (12).

2. Dispositif selon la revendication 1, **caractérisé en ce que** les moyens de marquage (19) émettent des ondes électromagnétiques ou acoustiques et le dispositif comprend en plus un détecteur de position (24), qui comprend dans l'espace au moins deux capteurs (23) détectant les ondes électromagnétiques ou acoustiques émises par les moyens de marquage (19).

3. Dispositif selon la revendication 1, **caractérisé en ce que** les moyens de marquage (19) détectent des ondes électromagnétiques ou acoustiques et le dispositif comprend en plus un détecteur de position (24), qui comprend dans l'espace au moins deux émetteurs (23) qui émettent les ondes électromagnétiques ou acoustiques devant être détectées par les moyens de marquage (19).

4. Dispositif selon l'une quelconque des revendications 1 à 3, **caractérisé en ce que** les moyens de marquage (19) sont disposés sur le pointeur (3) entre l'épaulement (17) et l'extrémité arrière (16) dans un plan (18) perpendiculaire à l'axe longitudinal (9).

5. Dispositif selon l'une quelconque des revendications 1 à 3, **caractérisé en ce que** les moyens de marquage (19) sont disposés sur le pointeur (3) entre l'épaulement (17) et l'extrémité arrière (16) dans un plan (18) renfermant l'axe longitudinal.
